# EUROPEAN PATENT SPECIFICATION

(11) **EP 2 599 460 B1**
(45) Date of publication and mention of the grant of the patent: **18.05.2016**
(21) Application number: 12194633.9
(22) Date of filing: 28.11.2012
(51) Int. Cl.: A61F 2/16

(54) **Intraocular lens injection system**
System zum Einsetzen einer Intraokularlinse
Système d'injection de lentille intraoculaire

(30) Priority: 29.11.2011 JP 2011260879
(43) Date of publication of application: 05.06.2013
(73) Proprietor: NIDEK CO., LTD, Gamagori-shi Aichi 443-0038 (JP)
(72) Inventor: Nagasaka, Shinji, Gamagori-shi, Aichi 443-0038 (JP); Sunada, Tsutomu, Gamagori-shi, Aichi 443-0038 (JP)
(74) Representative: Prüfer & Partner mbB Patentanwälte · Rechtsanwälte

(56) References cited:
- EP-A2- 1 790 317
- US-A1- 2003 212 406
- US-A1- 2011 082 463

## Description

### Field of the Invention

The present invention is related to an intraocular lens (IOL) injection instrument (injector) and an IOL injection system having a holder for holding the IOL injector.

### Related Art

There is an injector of preset type in which a soft IOL is stored in advance in the injector (JP2006-197994A). The preset-type injector has a problem that an impact applied during conveyance causes problems such as breakage of the IOL stored inside. In response to this, there is an IOL injection system for storing and conveying a preset-type injector held in a holder (JP2007-089915A).

From document US2003/212406A1, an intraocular lense injection system is known comprising an IOL injection instrument, a holder to hold the IOL injection instrument, whereby the IOL injection instrument includes a lense storage part to store IOL, and the holder includes a restriction member to restrict movement of a lense storage part. The restriction member has two arm portions inserted between a lense-holding member and a push member. The arm portions of the restriction member elastically hold the push member so that the restriction member cannot come off the push member unless external force is applied thereto. The restriction member can be moved so as to be removed between lense-holding member and the push member before the IOL is moved from a first or a standby position to the second or insertion position. The restriction member does not comprise side surfaces and a bottom surface surrounding the injection instrument and the restriction member does not comprise a columnar portion including columnar restriction portions extending from the bottom surface.

### Related Art Documents

### Patent Documents

Patent Document 1: JP2006-197994A
Patent Document 2: JP2007-089915A
Patent Document 3: US 2003/212406 A1

### SUMMARY OF INVENTION

### Problems to be Solved by the Invention

As well as the need of safely holding an IOL in an unused state, a preset-type injector is required to be released from a storage state with minimum processes for saving a doctor's time and effort and for keeping a sterilized state.

The present invention has been made to provide an IOL injection system for safely storing an IOL during storage and easily releasing the IOL from a storage position by a simple operation when used.

### Means of Solving the Problems

To solve the above problem, one aspect of the present invention provides an intraocular lens (IOL) injection system according to claim 1.

Further developments of the present invention are given in the dependent claims.

### BRIEF DESCRIPTION OF THE DRAWINGS

Fig. 1 is a perspective view of an intraocular lens (IOL) injection system;
Fig. 2 is an external perspective view of an injector and a holder;
Fig. 3A is a sectional view of the IOL injection system;
Fig. 3B is a sectional view of the injector;
Fig. 3C is a sectional view of the holder;
Fig. 4 is an explanatory view of a plunger;
Fig. 5 is an explanatory view of a configuration of a lens storage part;
Fig. 6A is a sectional view of the injector when an IOL is placed outside an axis;
Fig. 6B is a sectional view of the injector when the IOL is placed on the axis; and
Fig. 7 is a view of an IOL injection system according to a second embodiment.

### DESCRIPTION OF EMBODIMENTS

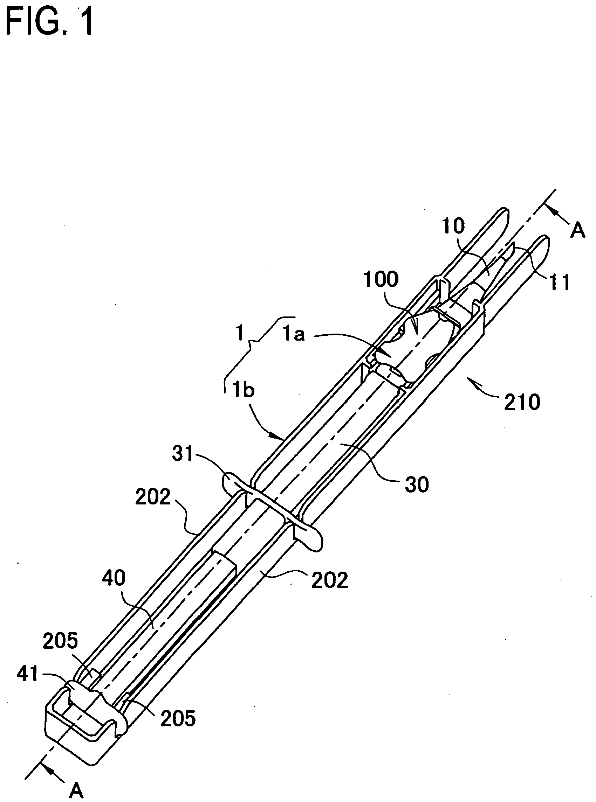

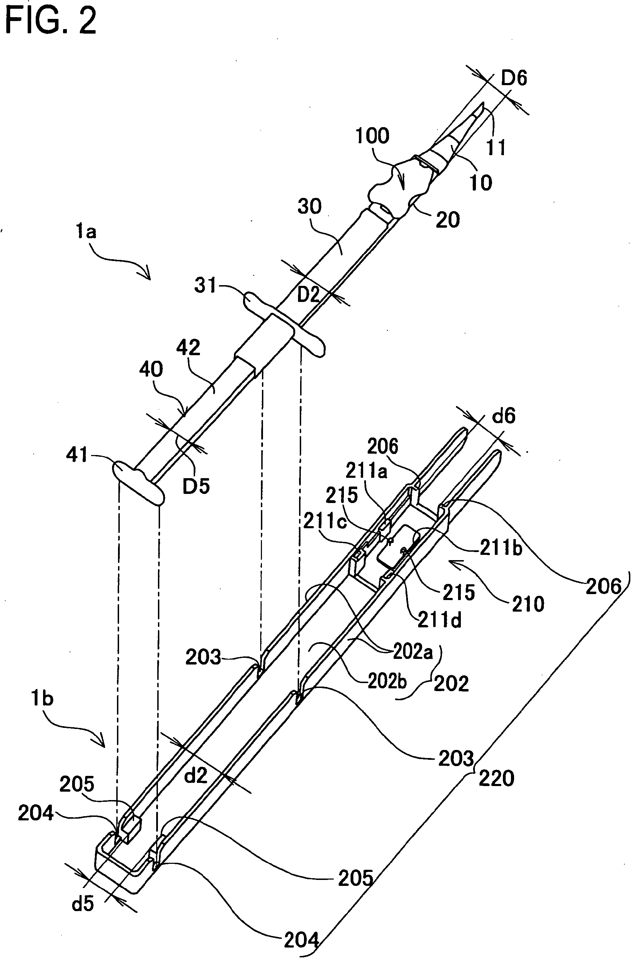

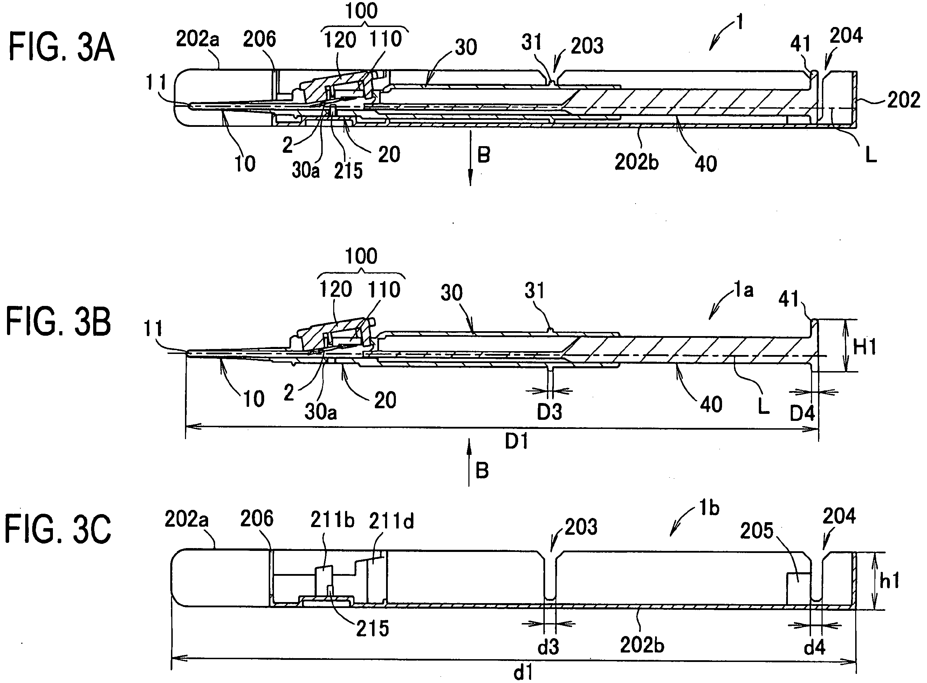

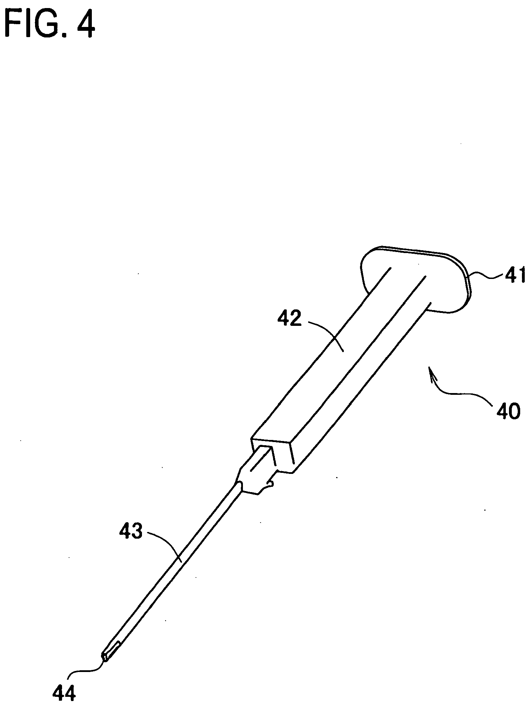

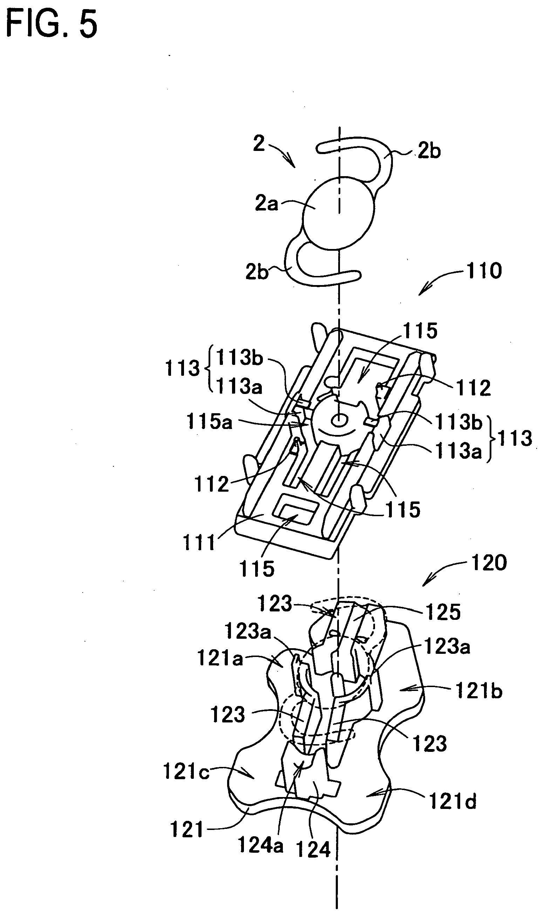

Fig. 1 is a perspective view of an IOL injection system 1 according to a present embodiment. Fig. 2 is a perspective view of an injector 1a and a holder 1b. Fig. 3A is a sectional view of the IOL injection system 1, taken along an arrow A-A in the IOL injection system 1 of Fig. 1. Fig. 3B is a sectional view of the injector 1a. Fig. 3C is a sectional view of the holder 1b. Fig. 4 is an explanatory view of a configuration of a plunger. Fig. 5 is an explanatory view of a configuration of a lens storage part.

The IOL injection system 1 includes the injector 1a and the holder 1b. The injector 1a is formed of a resin material and the like by molding or formed by cutting process by shaving a resin. The injector 1a is arranged to store an IOL 2 inside in an unused state and to fold and inject the IOL into an eye of a patient when used. The holder 1b is arranged to hold the injector 1a in an unused state.

The injector 1a includes a main body (a cylindrical portion) 30, a plunger 40, and a lens storage part 100. The main body 30 includes, on its front end side, an injection part 10 having a front end 11 and a setting part 20. The body 30 further includes, on its rear end side, a holding portion 31. This holding portion 31 can be held with the fingers of an operator to support the body 30.

The injection part 10 is of a cylindrical shape and has a region (an inner wall) having an inner diameter gradually decreasing (tapering) toward the front end 11. When passing through the injection part 10, the IOL 2 is folded into a smaller size along the inner wall of the injection part 10. The front end 11 is formed with a cutout (a bevel). This bevel serves to determine an opening direction of the IOL.

The setting part 20 is provided on a rear end of the injection part 10. In the setting part 20, there are located a setting surface on which the IOL 2 pushed out of the lens storage part 100 is to be placed and a space (clearance) defined by the setting surface and the inner wall of the main body 30. In the setting part 20 (at a holder 1b side) of the body 30, a through hole 30a is formed to allow a part of the holder 1b to pass through. The through hole 30a is provided to allow a part of the holder 1b to pass through and be positioned inside the body 30. This part of the holder 1b placed in the main body 30 serves to fix or support restriction portions 113 of the lens storage part 100 described later.

The lens storage part 100 serves as a lid member to close an opening (not assigned with a reference sign in the figure) of the setting part 20. Further, the lens storage part 100 includes a restriction means to restrict movement of the IOL 2 outside an axis L during storage of the injector 1a. The lens storage part 100 also includes a release means to release restriction of the IOL 2 by the restriction member outside the axis L and to bring the IOL 2 onto the axis L when the injector 1a is used. Detailed explanation for the restriction member and the release member is given thereafter.

The plunger 40 is moved back and forth along the axis L in a passage extending from the body 30 to the front end 11 of the injection part 10. The plunger 40 is formed with a press part 41, a basal part 42 connected to the press part 41, a push rod 43 connected to the basal part 42, and a tip portion 44 connected to a distal end of the push rod 43. When used, the IOL 2 placed on the setting part 20 is pressed by the tip portion 44 of the plunger 40, and thereby the IOL 2 is folded into a smaller size along the inner wall of the injection part 10.

The IOL 2 is stored in the lens storage part 100. As the IOL 2, a one-piece IOL having an optical part 2a and a pair of support parts 2b integrally made of soft material is used (see Fig. 5). The one-piece IOL is made from a single material such as HEMA (hydroxyethyl methacrylate), a composite material of acrylic ester and methacrylic acid ester, and so on. Other than this, the one-piece IOL is made from a soft IOL material which has been known. In the lens storage part 100, a foldable known IOL 2 can be stored. For example, a three-piece IOL having an optical part and support parts that are separately formed, a plate-type IOL having support parts of a plate-like shape, and others may be adopted.

In Fig. 5, the lens storage part 100 is configured with a holding part 110 to hold the unused IOL 2 and a release part 120 to release retention (restriction) of the IOL 2 by the holding part 110. During storage, the IOL 2 is retained without being subjected to any stress from a restriction member which will be described later. The state that the IOL 2 is not subjected to stress includes a state that the stress is applied to an extent that unintended deformation of the unused IOL 2 does not occur.

The holding part 110 includes a base 111, restriction portions 112 and 113, and through holes 115 and 115a. The restriction portions 112 and 113 are arranged to restrict movement of the IOL 2 placed on the base 111. The through holes 115 and 115a are formed on a part of the base 111 and arranged to pass a part of the release part 120.

The base 111 is formed to conform to an opening of the setting part 20 so as to close the opening. The restriction portions 112 and 113 are each formed in a columnar shape extending perpendicularly from the base 111 so that they extend perpendicularly into the body 30 when the lens storage part 100 is placed to close the setting part 20 (opening). The restriction portions 112 are provided in positions along (in contact with) an outer circumferential shape of the optical part 2a of the IOL 2 placed on the base 111. Thus, the IOL 2 placed on the base 111 is prevented from horizontally rotating or moving with respect to the base 111.

Each restriction portion 113 including a columnar portion 113a and an end portion 113b is formed to be located more outside than an outer peripheral edge of the optical part 2a placed on the base 111. The columnar portions 113a extending perpendicular to the base 111 prevent the optical part 2a from horizontally rotating and moving on the base 111. The end portions 113b are formed by bending a distal end of each columnar portion 113a inwardly (toward a center of the optical part 2a) and thus extend in a horizontal direction by a required distance. Each end portion 113b has a length long enough to come into contact with a part of the optical part 2a placed on the base 111 to restrict movement of the optical part 2a even when the optical part 2a attempts to move in a vertical direction ( in a direction to separate from the base 111). Specifically, the end portions 113b restrict the movement of the optical part 2a in a pushing direction of the release part 120.

The end portions 113b are not deformed by the weight or movement (slight movement) of the IOL 2, but each end portion 113b has a thickness having a predetermined flexibility and strength so that the end portions 113b are deformed by the pushing force applied by the release part 120. Thus, the end portions 113b are not deformed by the movement of the IOL 2 during storage but deformed by the pressure applied by the release part 120. The end portions 113b are deformed relative to connecting points (bending points) with the columnar portions 113a in a pushing direction of the release part 120 by the pressure applied from the release part 120. By the deformation of the end portions 113b, the restriction of the optical part 2a is released and the optical part 2a is allowed to be moved onto the axis L from outside of the axis L.

The through holes 115 are provided in positions corresponding to each portion (the optical part 2a and the support parts 2b) of the IOL 2 placed on the base 111 and a position corresponding to a centering portion 124 of the release part 120. The through holes 115 are formed on the base 111 as corresponding to each portion of the IOL 2, and thereby the IOL 2 can be pushed by the release part 120 that has passed the through holes 115 with well balanced. Further, the centering portion 124 is positioned on a pressing axis of the plunger 40 so that movement of the plunger 40 in an axial direction can be stabilized.

In the present embodiment, the through holes 115a are formed in the space defined by the optical part 2a and the columnar portions 113a so that a part of the release part 120 having passed the through holes 115a pushes and lifts the end portions 113b. The end portions 113b are thus pushed and deformed by the release part 120 in advance, so that the IOL 2 is moved onto the axis L without contacting with the end portions 113b.

The release part 120 includes a press part 121, a plurality of contact portions 123, and the centering portion 124. The press part 121 is a portion where an operator applies pressure by fingers and the like. The contact portions 123 are arranged to directly push the IOL 2 on the base 111 (outside the axis L) by the pushing force applied by the press part 121 and bring the IOL 2 onto the axis L. The centering portion 124 is a portion to prevent wobbling of the plunger 40.

The press part 121 of the present embodiment has contact regions 121 a to 121 d. The contact regions 121 a to 121 d are provided to make the press part 121 and a part of the holder (a restriction member 210 which is described later) interfere with each other when the press part 121 is pushed in the pushing direction while the injector 1a is retained by the holder 1b. To be specific, the contact regions 121a to 121d are formed by widening a part of the press part 121 to be wider than a width of the body 30 (setting part 20). The restriction member 210 of the holder 1b and the contact regions 121a to 121d of the injector 1a (the release part 120) interfere with each other during storage, so that the movement of the press part 121 in the pushing direction is restricted and the IOL 2 is prevented from being pushed onto the axis L by an error.

The plurality of contact portions 123 are each formed in a columnar shape extending from the press part 121 and each contact portion 123 has an end shape almost similar to (slightly smaller than) a shape of an opening of each through hole 115. Each contact portion 123 has a length long enough to come to contact with each portion of the IOL 2 that has passed through each through hole 115 and to push the IOL 2 onto the axis L when the whole release part 120 is pushed toward the holding part 110. The end portions of the contact portions 123 serve as a setting surface when the IOL 2 released from the restriction is placed on the axis L. Namely, a folding operation of the IOL 2 by the plunger 40 can be started easily without detaching the release part 120 from the body 30 after the release part 120 (press part 121) is pushed in the pushing direction.

Among the plurality of the contact portions 123, contact portions 123a corresponding to the through holes 115a are arranged to pass through the through holes 115a and come to contact with the end portions 113b. After the release part 120 (press part 121) is further pushed while the contact portions 123a are in contact with the end portions 113b, the end portions 113b are gradually press-widened outwardly by the pressing force of the contact portions 123a. Thereby, the IOL 2 is allowed to be moved onto the axis L from outside of the axis L.

A recess 124a is formed on an upper surface of the centering portion 124. The recess 124a has an almost similar shape with an outer shape of the plunger 40 and serves as a centering mechanism for guiding back and forth movement of the plunger 40 along the axis L when the IOL 2 is placed on the axis L. Further, on ends (upper surfaces) of the contact portions 123 corresponding to the support parts 2b, grooves 125 having almost similar shape with the outer shape of the plunger 40 (push rod 43) are formed extending in a front to rear direction (in a direction of a pressing axis) of the IOL 2 to serve as the centering mechanism of the plunger 40.

According to the above configuration, the IOL 2 is retained by the restriction portions 112 and 113 of the holding part 110 without being subjected to stress. Further, by the interference of the restriction member 210 of the holder 1b and the release part 120, the storage state of the IOL 2 is stably maintained. On the other hand, the restriction member 210 is separated from the release part 120 and the restriction of the release part 120 is released when the injector 1a is lifted from the holder 1b when used. Then, by pushing the release part 120 which is released from the restriction, the IOL 2 is allowed to be moved from a storage position (outside the axis L) to a use position (on the axis L).

The holder 1b includes a wall 202 (side surfaces 202a and a bottom surface 202b) surrounding the injector 1a, a misalignment prevention mechanism 220 restricting movement of the injector 1a in the front to rear direction (axial direction), and the restriction member 210 restricting the movement of the lens storage part 100 inside the wall 202.

A distance d1 in a front to rear direction of the side surfaces 202a of the wall 202 is longer than a whole length D1 of the injector 1a. A distance d2 of the facing side surfaces 202a is longer than a width D2 of the main body 30. A depth (distance from an upper end of the side surface 202a to the bottom surface 202b) h1 of the wall 202 is similar to or deeper than a maximum value H1 of a height of the injector 1a. By the above described wall 202 (the side surfaces 202a and the bottom surface 202b) of the holder 1b, the injector 1a as a whole is surrounded to be prevented from being directly subjected to the stress (impact) from outside during storage.

The misalignment prevention mechanism 220 is provided in plural portions on the wall 202 and inside the wall 202, the mechanism 220 including grooves 203 and 204 formed by cutting out a part of the wall 202 in a depth direction and holding portions 205 and 206 provided inside the holder 1b.

The grooves 203 are formed in positions corresponding to the holding portion 31 of the injector 1a. A width d3 of each groove 203 is almost equal to (or slightly wider than) a thickness D3 of the holding portion 31. The holding portion 31 is placed on the grooves 203 so that the holder 1b and the holding portion 31 interfere with each other in positions of the grooves 203, and thereby movement of the body 30 in the front to rear (axial) direction inside the holder 1b is prevented.

The grooves 204 are formed in positions corresponding to the press part 41 of the plunger 40. A width d4 of each groove 204 is almost equal to (or slightly wider than) a thickness D4 of the press part 41. The press part 41 is placed on the grooves 204 so that the holder 1b and the press part 41 interfere with each other in the positions of the grooves 204, and thereby movement of the plunger 40 inside the holder 1b in the front to rear direction is prevented.

The holding portions 205 are columnar portions extending from the bottom surface 202b and provided at the rear (rear end side) of the holder 1b (the side surface 202a). Specifically, the holding portions 205 are provided inside the side surfaces 202a in positions corresponding to a connecting position of the press part 41 and the basal part 42. An interval d5 of the facing holding portions 205 is almost equal to a width D5 of the basal part 42 so that the basal part 42 is held by the holding portions 205. By holding the basal part 42 by the holding portions 205, the movement of the plunger 40 in a left and right (width) direction inside the holder 1b is prevented.

The holding portions 206 are columnar portions extending from the bottom surface 202b and provided in front (front end side) of the holder 1b (side surface 202a). Specifically, the holding portions 206 are provided inside the side surfaces 202a in positions corresponding to the injection part 10 retained by the holder 1b. An interval d6 of the facing holding portions 206 is almost equal to a diameter D6 of the injection part 10 at a position corresponding to the holding portions 206 so that the tapered injection part 10 is held by the holding portions 206. Thereby, the injector 1a is prevented from moving toward the front end 11 inside the holder 1b.

The restriction member 210 is a columnar portion extending from the bottom surface 202b, including columnar restriction portions 211 a to 211 d attached to an inside of the side surfaces 202a and columnar support portions 215 attached to the bottom surface 202b.

The restriction portions 211a to 211d are provided in positions corresponding to the contact regions 121a to 121d of the lens storage part 100 of the injector 1a retained by the holder 1b, respectively, to restrict movement of the release part 120 in the pushing direction. Herein, an interval of the facing restriction portions 211a and 211 b and an interval of the facing restriction portions 211c and 211d are each formed almost equal to a width of the setting part 20. Thereby, the setting part 20 is held by the restriction portions 211a to 211d, and movement (misalignment) of the injector 1a in the left and right direction is prevented.

The columnar restriction portions 211 a to 211d have heights high enough to be in contact with the contact regions 121 a to 121 d of the press part 121 (sides facing the holder 1b), respectively, while the injector 1a is retained by the holder 1b. In the present embodiment, the press part 121 in the storage state is attached to the body 30 obliquely as a slant surface to be away from the pressing axis L from the front end 11 toward the rear end (the press part 41). In accordance with this configuration, the restriction portions 211c and 211d at the rear end side have the heights higher than that of the restriction portions 211a and 211 b at the front end 11 side.

The heights of the restriction portions 211 a to 211 d are determined to conform to the slant surface of the press part 121, and thereby each of the contact regions 121a to 121 d is evenly brought into contact with each of the restriction portions 211 a to 211d during storage and thus uniformly supported.

The columnar support portions 215 are formed in a position corresponding to the through hole 30a of the setting part 20. Each support portion 215 has a length (height) long enough to be in contact with the end portions 113b restricting the IOL 2 while the injector 1a is retained by the holder 1b. Owing to the support of the end portions 113b by the support portions 215, the retention of the IOL 2 by the restriction portions 113 is maintained even when the IOL 2 is moved during storage and the force to outwardly open the end portions 113b is applied.

In this manner, the movement of the lens storage part 100 in the pushing direction is restricted by the restriction portions 211a to 211d provided in the holder 1b, and thereby it is prevented that the storage of the IOL 2 is released by an error during storage. Further, the movement of the lens storage part 100 in the pushing direction is restricted by a simple structure of bringing the lens storage part 100 to be in contact with the restriction member 210. Furthermore, when the injector 1a is used, the restriction by the restriction member 210 can be easily released only by detaching the injector 1a from the holder 1b.

Next, an operation of injecting an IOL into an eye by using the IOL injection system having the above configuration is explained.

As shown in Fig. 1, during the storage of the injector 1a (IOL 2), each component of the injector 1a is attached to the misalignment prevention mechanism 220 of the holder 1b so that the movement of the injector 1a inside the holder 1b in the front to rear direction and the left and right direction is prevented. The movement of the release part 120 (press part 121) in the pushing direction is restricted by the contact regions 121a to 121 d of the lens storage part 100 contacting with the restriction portions 211a to 211d. Further, the support portions 215 are arranged to pass through the through hole 30a of the setting part 20 and come to contact with the end portions 113b so that the movement of the end portions 113b in the opening direction is prevented. The IOL injection system 1 during storage is hermetically sealed and sterilized in a case or a bag which is omitted with illustration in the figure.

Inside the injector 1a, an opening of the setting part 20 is closed by the lens storage part 100 as a lid member to store the IOL 2 inside. In this state, the IOL 2 is not subjected to stress by the restriction portions 112 and 113 and restricted from moving on the base 111. The end portions 113b are supported by the support portions 215 of the holder 1b.

In use of the injector 1a, the IOL injection system 1 is taken out of the not-shown case or bag. Then, as shown in Fig. 3B, an operator lifts the injector 1a to be separated from the holder 1b (in a direction indicated with an arrow B).

By this operation, the holding portion 31 is separated from the grooves 203 as the misalignment prevention mechanism 220 and the press part 41 is separated from the grooves 204, so that the plunger 40 is allowed to move in the front to rear direction. Each of the contact regions 121 a to 121 d of the press part 121 is separated from each of the restriction portions 211a to 211d of the holder 1b, allowing the press part 121 to be pushed toward the body 30. Further, the support portions 215 are separated from the end portions 113b, allowing the end portions 113b to be deformed outwardly.

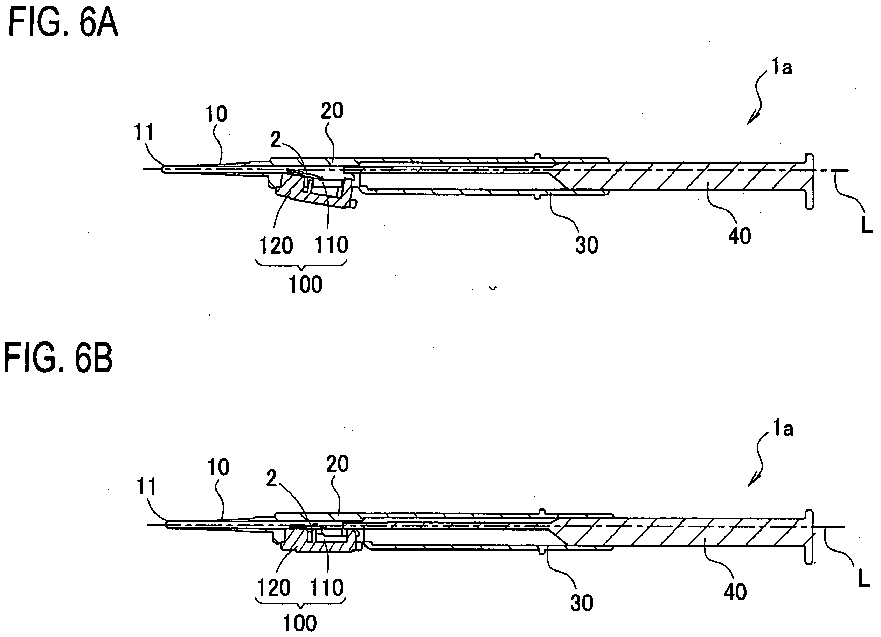

After the injector 1a is taken out of the holder 1b, the operator releases the restriction of the IOL 2 by the lens storage part 100. Figs. 6A and 6B are sectional views of the side surface of the injector 1a after detached from the holder 1b. Fig. 6A shows a state that the IOL 2 is stored by the lens storage part 100 outside the axis L. Fig. 6B shows a state that the IOL 2 is placed on the axis L after released from the storage by the lens storage part 100.

Since a part of the lens storage part 100 serves as the setting surface of the IOL 2 in the present embodiment, Figs. 6A and 6B show the injector 1a placed upside down from the state shown in Fig. 3B with the lens storage part 100 being located in a lower side in the figure.

When the press part 121 of the release part 120 is pushed in the storage state in Fig. 6A, the contact portions 123a having passed the through holes 115a firstly come to contact with the end portions 113b. The end portions 113b are deformed outwardly by the pressure applied from the press part 121. The press part 121 is further pressed, and each contact portion 123 having passed each through hole 115 comes to contact with the IOL 2.

As shown in Fig. 6B, after the whole release part 120 is pressed, the IOL 2 is placed on the setting surface configured by the ends of the contact portions 123. By utilizing the release part 120 as the setting surface, a clearance between the IOL 2 and the setting surface is rarely created, so that problems such as the plunger getting onto the optical part 2a or getting under the optical part 2a can be prevented.

After the IOL 2 is placed on the setting surface (on the axis L), the operator rotates the injector 1a such that an orientation of the bevel of the front end 11 is appropriately aligned. Then, a known viscoelastic substance is injected in the injection part 10 from a not-shown opening or the front end 11.

The operator presses the press part 41 to move the plunger 40 along the recess 124a and the groove 125 as the centering mechanism in the front and rear direction along the axis. The press part 41 is further pressed after the tip portion 44 comes to contact with a peripheral edge (edge) of the optical part 2a, and the IOL 2 is moved toward the front end 11 and folded along the inner wall of the injection part 10.

By further pressing the press part 41, the folded IOL 2 is injected into an eye from the front end 11. Then, the IOL 2 returns to its unfolded original shape by the restoring force in a capsule. After the pair of support parts 2b is positioned along the capsule, the optical part 2a is retained by stress applied inside the eye.

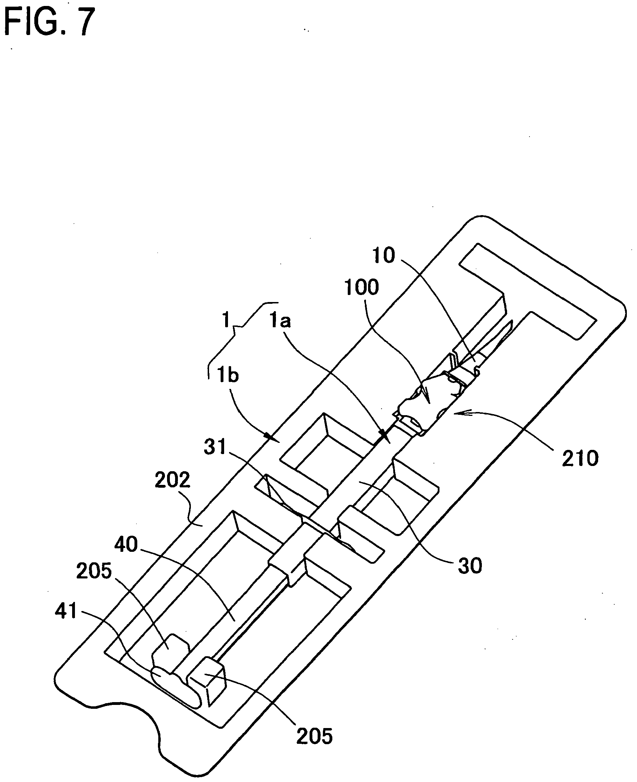

The present invention is not limited to the above mentioned configuration. For example, as shown in a second embodiment of Fig. 7, the holder 1b may be formed to surround the whole body of the injector 1a including the holding portion 31 and the press part 41. This configuration facilitates absorbing the impact applied to the injector 1a by the holder 1b. In a case that the restriction member 210 has a complicated shape, the holder 1b and the restriction member 210 may be formed separately and assembled together by such as bonding, welding, or fitting of a recess and a protrusion.

Also, the restriction member 210 and the holder 1b may be formed with a recess and a protrusion to be engaged together so that the restriction member 210 remains to be attached to the injector 1a when the injector 1a is detached from the holder 1b. By this configuration, an operator can control a timing of releasing the restriction by the restriction member 210. For instance, the restriction by the restriction member 210 may be released just before the use of the injector 1a.

In the above embodiment, an example of the IOL 2 stored outside the axis L is explained. Alternatively, an injector having the IOL 2 stored on the axis L may also be provided with a restriction member in a moving direction of a lens storage part to distinguish a storage state and a use state of the IOL 2.

To be specific, the IOL 2 is placed on the axis L inside the injector 1a (injection part 10), and a restriction part for restricting the movement of the IOL 2 is provided in the moving direction (at the front end 11 side) of the IOL 2. The restriction part is deformed toward the front end 11 (in the moving direction of the IOL 2) by the stress applied by the forward movement of the plunger. Namely, the restriction part is directly pressed by the plunger 40 and deformed in the moving direction, or the restriction part is pushed via the IOL 2 that is pressed by the plunger 40 and deformed in the moving direction. According to this configuration, the movement of the IOL 2 placed on the axis L is restricted by the restriction part during storage. In use, the plunger 40 is pressed toward the front end 11 and the restriction part is deformed in the moving direction by the pressing force directly applied by the plunger 40 (or the pressing force indirectly applied via the IOL 2), and thus the restriction of the IOL 2 by the restriction part is released. Then, the plunger 40 is kept to be pressed toward the front end 11, and the IOL 2 is folded into a smaller size inside the injection part 10 and injected into the eye from the front end 11.

## Claims

1. An IOL injection system including: an IOL injection instrument (1a) having an axis (L); and a holder (1b) to hold the IOL injection instrument (1a), the IOL injection instrument (1a) includes a lens storage part (100) to store an IOL (2), said lens storage part (100) being configured with a holding part (110) to hold the unused IOL (2) and a release part (120) to release restriction of the IOL (2) by the holding part (110), said release part (120) being attached to the holding part and allowed to be pushed in the holding part, and the holder (1b) includes side surfaces (202a) and a bottom surface (202b) surrounding the injection instrument (1), and a restriction member (210) to restrict movement of said lens storage part (100), said restriction member (210) having a columnar portion including columnar restriction portions (211 a to d) extending from said bottom surface (202b), wherein the movement of the release part (120) in the pushing direction is restricted by contact regions (121 a to d) of the lens storage part (100) contacting with restriction portions (211a to d), and wherein the restriction by the restriction member (210) can be released only by detaching the injector (1a) from the holder (1b).

2. The IOL injection system (1) according to claim 1, wherein
the holding part (110) includes an end portion (113b) positioned in a moving direction of the release part (120) to restrict movement of the IOL (2) during storage and to be opened in the pushing direction by pressure applied by the release part,
the restriction member (210) includes a support portion (215) positioned in a deforming direction of the end portion (113b) to prevent deformation of the end portion in the pushing direction,
the support portion (215) is in contact with the end portion (113b) to restrict the deformation of the end portion in the pushing direction while the IOL injection instrument (1a) is retained by the holder (1b), and
the support portion (215) is separated from the end portion (113b) when the IOL injection instrument (1a) is detached from the holder (1b) so that restriction of the end portion by the support portion is released.

3. The IOL injection system (1) according to any one of claims 1 to 2, wherein the IOL injection instrument (1a) includes:
an injection part (10) having an inner wall to fold the IOL (2) and a front end (11) to inject the IOL into an eye through an incision of the eye; and
a press member (40) to be moved back and forth along an axis (L) to inject the IOL (2) placed on the axis (L) from the front end (11).

## Patentansprüche

1. Injektionssystem für eine Intraokularlinse, welches beinhaltet: ein Injektionsinstrument (1a) für eine Intraokularlinse, welches eine Achse (L) besitzt; und einen Halter (1b), um das Injektionsinstrument (1a) für eine Intraokularlinse zu halten, wobei das Injektionsinstrument (1a) für eine Intraokularlinse ein Linsenlagerteil (100) besitzt, um eine Intraokularlinse (2) zu lagern, wobei das Linsenlagerteil (100) mit einem Halteteil (110), um die unbenutzte Intraokularlinse (2) zu halten, und einem Abgabeteil (120), um die Beschränkung der Intraokularlinse (2) durch das Halteteil (110) zu lösen, ausgestaltet ist, wobei das Abgabeteil (120) am Halteteil angebracht ist und es möglich ist, in das Halteteil gedrückt zu werden, und der Halter (1b) Seitenoberflächen (202a) und eine Bodenoberfläche (202b), welche das Injektionsinstrument (1) umgeben, beinhaltet, und ein Begrenzungsteil (210), um die Bewegung des Linsenlagerteils (100) zu beschränken, wobei das Begrenzungsteil (210) einen säulenförmigen Abschnitt besitzt, welcher säulenförmige Begrenzungsabschnitte (211a bis d) besitzt, welche von der Bodenoberfläche (202b) ausgehen,
wobei die Bewegung des Abgabeteils (120) in der Stoßrichtung durch Kontaktbereiche (121a bis d) des Linsenlagerteils (100) beschränkt ist, welches mit Begrenzungsabschnitten (211a bis d) in Kontakt steht, und wobei die Beschränkung durch das Begrenzungsteil (210) nur durch ein Lösen des Injektors (1a) vom Halter (1b) möglich ist.

2. Injektionssystem für eine Intraokularlinse gemäß Anspruch 1,
wobei das Halteteil (110) einen Endabschnitt (113b) beinhaltet, welcher in einer Bewegungsrichtung des Löseteils (120) positioniert ist, um die Bewegung der Intraokularlinse (2) während des Lagerns zu beschränken und um in einer Stoßrichtung durch Druck, welcher durch das Löseteil aufgebracht wird, geöffnet zu werden,
wobei das Begrenzungsteil (210) einen Lagerabschnitt (215) beinhaltet, welcher in einer Deformationsrichtung des Endabschnitts (113b) positioniert ist, um eine Deformation des Endabschnitts in der Stoßrichtung zu verhindern,
wobei der Lagerabschnitt (215) in Kontakt mit dem Endabschnitt (113b) ist, um die Deformation des Endabschnitts in der Stoßrichtung zu beschränken, während das Injektionsinstrument (1a) für eine Intraokularlinse durch den Halter (1b) zurückgehalten wird, und
wobei der Lagerabschnitt (215) vom Endabschnitt (113b) getrennt ist, wenn das Injektionsinstrument (1a) für eine Intraokularlinse vom Halter (1b) gelöst wird, so dass die Beschränkung des Endabschnitts durch den Lagerabschnitt gelöst wird.

3. Injektionssystem (1) für eine Intraokularlinse gemäß einem der Ansprüche 1 bis 2, wobei das Injektionsinstrument (1a) für eine Intraokularlinse beinhaltet:
ein Injektionsteil (10), welches eine innere Wand besitzt, um die Intraokularlinse (2) und ein vorderes Ende (11) zu falten, um die Intraokularlinse in ein Auge durch einen Schnitt im Auge einzuführen; und
ein Druckteil (40), welches vor und zurück entlang einer Achse (L) bewegt werden kann, um die Intraokularlinse (2), welche auf der Achse (L) positioniert ist, von dem vorderen Ende (11) einzuführen.

## Revendications

1. Système d'injection d'une lentille intraoculaire IOL comprenant un instrument d'injection d'une IOL (1a) ayant un axe (L) et un support (1b) destiné à maintenir l'instrument d'injection d'une IOL (1a), cet instrument d'injection d'une IOL (1a) comprenant une partie de stockage de lentille (100) destinée à stocker une IOL (2), cette partie de stockage de lentille (100) étant conformée avec une partie-support (110) destinée à maintenir l'IOL (2) non utilisée et une partie de libération (120) permettant de libérer la retenue de l'IOL (2) par la partie support (110), cette partie de libération (120) étant fixée à la partie support et susceptible d'être poussée dans cette partie support, et le support (1b) comprenant des surfaces latérales (202a) et une surface de fond (202b) entourant l'instrument d'injection (1), ainsi qu'un élément de retenue (210) permettant de limiter le déplacement de la partie de stockage de lentille (100), cet élément de retenue (210) ayant une partie en forme de colonne comprenant des parties de retenue en forme de colonnes (211a à d) s'étendant à partir de la surface de fond (202b),
le déplacement de la partie de libération (120) dans la direction de poussée étant limité par des régions de contact (121a à d) de la partie de stockage de lentille (100) venant en contact avec des parties de retenue (211a à d), et la retenue par l'élément de retenue (210) ne pouvant être libérée qu'en séparant l'injecteur (1a) du support (1b).

2. Système d'injection d'une IOL (1) conforme à la revendication 1, dans lequel :
la partie support (110) comprend une partie d'extrémité (113b) située dans la direction de déplacement de la partie de libération (120) destinée à limiter le déplacement de l'IOL (2) au cours du stockage et destinée à être ouverte dans la direction de poussée par une pression appliquée par la partie de libération,
l'élément de retenue (210) comprenant une partie support (215) située dans une direction de déformation de la partie d'extrémité (113b) pour empêcher la déformation de la partie d'extrémité dans la direction de poussée,
la partie support (215) étant en contact avec la partie d'extrémité (113b) pour limiter la déformation de la partie d'extrémité dans la direction de poussée lorsque l'instrument d'injection d'une IOL (1a) est maintenu par le support (1b), et
la partie support (215) étant séparée de la partie d'extrémité (113b) lorsque l'instrument d'injection d'une IOL (1a) est séparé de l'organe de fixation (1b) de sorte que la retenue de la partie d'extrémité par la partie support soit libérée.

3. Système d'injection d'une IOL (1) conforme à l'une quelconque des revendications 1 et 2,
dans lequel l'instrument d'injection d'une IOL (1a) comprend :
une partie d'injection (10) ayant une paroi interne permettant de plier l'IOL (2) et une extrémité frontale (11) permettant d'injecter l'IOL dans un oeil par une incision réalisée dans cet oeil, et
un élément de compression (40) destiné à être déplacé en va et vient le long d'un axe (L) pour injecter l'IOL (2) située sur l'axe (L) à partir de l'extrémité frontale (11).
